# EUROPEAN PATENT APPLICATION

(11) **EP 0 727 484 A1**
(43) Date of publication of application: **21.08.1996**
(21) Application number: 96102188.8
(22) Date of filing: 14.02.1996
(51) Int. Cl.: C12N 7/00

(54) **Process for growing virus**

(30) Priority: 15.02.1995 JP 51737/95
(71) Applicant: Juridical Foundation The Chemo-Sero-Therapeutic Research Institute, Kumamoto-shi Kumamoto-ken (JP)
(72) Inventor: Ezoe, Shinsuke, Kumamoto-shi, Kumamoto-ken (JP); Uno, Katsuaki, Kumamoto-shi, Kumamoto-ken (JP); Ginnaga, Akihiro, Kumamoto-shi, Kumamoto-ken (JP); Fujikawa, Hideo, Kumamoto-shi, Kumamoto-ken (JP)
(74) Representative: VOSSIUS & PARTNER

(57) **Abstract**

A process for growing a virus comprising infecting chick embryo (CE) primary culture cells with said virus and culturing said cells by a suspension culture in a culture medium without adhesion of the cells to a solid phase. In accordance with the process of the present invention, a large amount of the desired virus can easily be obtained by a simple procedure without using a conventional solid phase such as a culture bottle, a hollow fiber or a microcarrier at a low cost.

## Description

The present invention relates to a method for growing a virus. More particularly, the present invention relates to a method for growing a virus by infecting chick embryo (CE) primary culture cells with said virus and culturing said cells by a suspension culture in a culture medium without adhesion of the cells on a solid phase such as a culture bottle, a hollow fiber or a microcarrier. The process of the present invention is useful for producing a large amount of the desired virus by a simple procedure without using a conventional solid phase such as a culture bottle, a hollow fiber or a microcarrier at a low cost.

Tissue culture cells have commonly been used for any fundamental experimental procedure such as isolation, identification, measurement of amount and purification of an animal virus. A suspension culture wherein the culture is conducted by suspending cells or virus-infected cells in a spinner flask, or in a culture tank for preparing an increased amount of viruses, is preferable for obtaining a large amount of cells or viruses (Mammalian Cell Culture Technology, P1-18, 1985, SOFT SCIENCE PUBLICATIONS).

However, use of a suspension culture is limited to only a few established cell lines (e.g. CHO cell, HmLu-1 cell, BHK-21 cell, cancer cell, various hybridoma cells), and primary culture cells derived from the living body cannot grow without being adhered to a solid phase except special cells such as hematopoietic cells.

Since viruses can only be viable and grow in a living cell, in order to culture adhesion-dependent primary culture cells or to prepare viruses sensitive to these cells, a wall of a vessel such as glass or plastic vessels as a solid phase had to be used. For the purpose of producing a large amount of cells or viruses, a solid phase such a hollow fiber (Science 178, p65-67, 1972) or a microcarrier (Nature 216, p64-65, 1967) has hitherto been used in order to enlarge the surface to which cells are adhered, and thereby the cells can be cultured on the enlarged surface.

Chick embryo (CE) primary culture cells are one of culture cells which have widely been used for growth and preparation of various viruses. Primary culture cells derived from CE or swine kidney cells are adhesion-dependent cells, and hence, cannot grow in a suspension culture. Accordingly, a culture glass bottle (e.g. Roux bottle, roller bottle) or a support such as the above-mentioned hollow fiber or microcarrier for production of a large amount of cells has been used, thereby cells are cultured while being adhered to a surface of such vessel.

However, the conventional procedure for culturing cells with the use of plenty of the above-mentioned glass vessels or the like is disadvantageous in that it is troublesome in handling and due to the possibility of contamination by bacteria. Besides, although it is preferable to use a tank for the purpose of producing a large amount of viruses, it is further disadvantageous from an economical point of view in addition to the troublesome procedure because of the necessity of having to use an expensive hollow fiber or microcarrier.

It is therefore the object of the present invention to provide a process for propagating viruses without the defects as mentioned above. The present inventors have found that a virus can be grown in CE primary culture cells by a suspension culture without being adhered to a solid phase, notwithstanding common knowledge that CE primary culture cells are an adhesion-dependent cell. That is, the present inventors have tried to prepare various viruses by culturing CE primary culture cells infected with said viruses in a suspension culture, and as a result, have found that the viruses grow well even in the CE primary culture cells by a suspension culture although the CE primary culture cells themselves did not grow. Accordingly, for growth of viruses, it has been found that a support is not always necessary, and hence, the process for preparing viruses of the present invention is more advantageous than a conventional culture using a solid phase to which cells are adhered.

Thus, the present invention is directed to a method for growing a virus by infecting chick embryo (CE) primary culture cells with said virus and culturing said cells by a suspension culture in a culture medium without adhesion of the cells to a solid phase. In accordance with the present invention, viruses can be prepared in a suspension culture using a large scale culture tank.

CE primary culture cells used herein may be any CE primary culture cell which can be obtained by conventionally known processes for preparation (cf. Experimental Virology, revised 2nd ed., Introduction, p 146-147, 1973, Maruzen).

Viruses grown in CE primary culture cells may be any virus which can infect CE or CE primary culture cells and are selected from the group consisting of infectious bursal disease virus (IBDV), avian reovirus (ARV), pigeon pox virus (PPV), fowl pox virus (FPV), avian infectious laryngotracheitis virus (ILTV), turkey herpesvirus, Marek's disease virus, Newcastle disease virus, avian infectious bronchitis virus, egg drop syndrome-1976 (EDS-76) virus, Ibaraki virus, bovine parainfluenza type 3 virus, rabies virus, rubella virus, mumps virus and influenza virus. Tissue culture solutions for culture of CE primary culture cells may be those commonly used for tissue cultures such as Eagle MEM medium.

Prior to infection with viruses, cells of CE of 7 to 14 days old, preferably 9 to 11 days old, animals are treated with 0.25% trypsin and then adjusted to 1 x 10⁴ to 3 x 10⁶, preferably 1 x 10⁵ to 2 x 10⁵, cells per 1 ml of a culture medium, which is then infected with a virus in an amount which optimizes the propagation of the virus and which may vary depending on the kind of viruses used. The culture medium may be supplemented with 30% or less, preferably 5 to 10% of a calf, chick or goat serum.

The culture of the cells is usually conducted by a tissue culture in a vessel such as a spinner flask or a fermenter conventionally used for culture of bacteria or established cell lines such as hybridoma cells, at 30 to 40°C, preferably at 32 to 38°C, for 2 to 8 days with stirring while the culture conditions are adjusted to hydrogen ion concentration (pH) of 5.0 to 9.5, preferably 6.5 to 7.6, and dissolved oxygen (DO) of 0.5 to 6.0 ppm, preferably 2 to 5 ppm.

The culture solution containing the grown viruses as prepared above is used for preparing various live vaccines or inactivated vaccines by the known procedures.

The present invention is illustrated in more detail by means of the following Examples but should not be construed to be limited thereto.

### Example 1

CE primary culture cells were suspended in Eagle's MEM medium (pH 7.2) supplemented with 5% calf serum at a concentration of 1 x 10⁵ cells per 1 ml of the culture medium. The resulting culture medium was mixed with attenuated IBDV-K strain at a multiplicity of infection (m.o.i.) of 0.00005 and adjusted to a total of 100 liters. A suspension culture was conducted in a 200 liter fermenter at 37°C for 6 days with stirring while the culture conditions were adjusted to pH 7.2 and DO of 3 ppm to allow for growth of IBDV. Every day during culture, 100 ml of the culture were sampled and a culture solution was obtained after removal of cells with a 1 µm filter.

A viral content of these culture solutions was measured by TCID₅₀ (50% tissue culture infectious dose) using CE cells where those cells showing cytopathic effect (CPE) were considered to be infected with viruses. The results are shown in Table 1.

Using the culture solution on Day 6, live and inactivated vaccines were prepared. A live vaccine was prepared by adding 5% (v/v) of lactose to the culture solution followed by lyophilization. An inactivated vaccine was prepared by treating the culture solution with 0.2% (v/v) of formalin at 37°C for 8 days for inactivation of the viruses and adding an equivalent amount of aluminum hydroxide gel (as aluminum chloride, 0.25% (w/v)). A titer of the prepared vaccines was measured against chicken. The live vaccine dissolved in phosphate buffered saline (PBS) was orally administered to 1 day old specific pathogen free (SPF) chicken at 10^{4.0} TCID₅₀/0.2 ml and immunization was conducted for 3 weeks. The inactivated vaccine (0.5 ml) was administered through intramuscular injection to 33 days old SPF chicken and immunization was conducted for 4 weeks. After immunization, a blood sample was taken and the titer of neutralization antibody, i.e.the maximum dilution which reduces the number of plaques by 50%, was measured. The results are shown in Table 2.

The viral content and the titer against chicken were measured in accordance with "Standard for Biological Preparation for Animals" (published by the Association of Biological Preparation for Animals on June 1993, page 178-183, 184-188).

As is clear from the results shown in Table 2, the IBDV live or inactivated vaccines prepared by suspension culture of IBDV-CE in accordance with the present invention exhibited a sufficient titer against chicken.

**Table 1**

| Viral Growth in IBDV-CE Suspension Culture | | | | | | | |
|---|---|---|---|---|---|---|---|
| | Viral Content (Log₁₀ TCID₅₀/ml) | | | | | | |
| Suspension Culture | 0 | 1 | 2 | 3 | 4 | 5 | 6 (Day) |
| | 2.0 | 6.5 | 8.6 | 8.5 | 8.5 | 8.5 | 8.6 |

**Table 2**

| Titer of IBDV Live and Inactivated Vaccines against Chicken | | | | | | | | | | |
|---|---|---|---|---|---|---|---|---|---|---|
| Vaccine | Number of Chicken | Titer of Neutralization Antibody | | | | | | | | |
| | | <512 | 512 | 1024 | 2048 | 4096 | 8192 | 16384 | 32768 | 65536 |
| Live¹⁾ | 10 | 0 | 1 | 4 | 3 | 2 | 0 | 0 | 0 | 0 |
| Inactivated²⁾ | 10 | 0 | 0 | 0 | 0 | 1 | 3 | 1 | 4 | 1 |

| | | | | | | | | | | |
|---|---|---|---|---|---|---|---|---|---|---|
| (Note) 1): Titer of neutralization antibody in control was less than 10 times. | | | | | | | | | | |
| 2) Titer of neutralization antibody in control was less than 4 times. | | | | | | | | | | |

### Example 2

CE primary culture cells were suspended in Eagle's MEM medium (pH 7.2) supplemented with 5% calf serum at a concentration of 1 x 10⁵ cells per 1 ml of the culture medium. The resulting culture medium was mixed with ARV58-132E50 strain at m.o.i. of 0.000006 and adjusted to 100 liters. A suspension culture was conducted in a 200 liter fermenter at 37°C for 6 days with stirring while the culture conditions were adjusted to pH 7.2 and DO of 3 ppm to allow for growth of ARV. Every day during culture, 100 ml of the culture were sampled and a culture solution was obtained after removal of cells with a 1 µm filter.

A viral content of these culture solutions was measured by plaque forming unit (PFU) using CE cells where those cells showing plaque were considered to be infected with viruses. The results are shown in Table 3.

Using the culture solution on Day 6, inactivated vaccines were prepared by treating the culture solution with 0.2% (v/v) of formalin at 37°C for 8 days for inactivation of the viruses and adding an equivalent amount of aluminum hydroxide gel (as aluminum chloride, 0.25% (w/v)). The titer of the prepared vaccine was measured against chicken. The inactivated vaccine (0.5 ml) was administered by intramuscular injection to 33 days old SPF chicken and immunization was conducted for 4 weeks. After immunization, a blood sample was taken and the titer of neutralization antibody, i.e. the maximum dilution which reduces the number of plaques by 90%, was measured using chicken kidney (CK) cells. The results are shown in Table 4.

The viral content and the titer against chicken were measured in accordance with "Standard for Biological Preparation for Animals" (published by the Association of Biological Preparation for Animals on June 1993, page 184-188).

As is clear from the results shown in Table 4, the ARV inactivated vaccines prepared by suspension culture of ARV-CE in accordance with the present invention exhibited a sufficient titer against chicken.

**Table 3**

| Viral Growth in ARV-CE Suspension Culture | | | | | | | |
|---|---|---|---|---|---|---|---|
| | Viral Content (Log₁₀ PFU₅₀/ml) | | | | | | |
| Suspension Culture | 0 | 1 | 2 | 3 | 4 | 5 | 6 (Day) |
| | <1 | 4.2 | 7.2 | 8.3 | 8.3 | 8.6 | 8.5 |

**Table 4**

| Titer of ARD Inactivated Vaccines against Chicken | | | | | | | |
|---|---|---|---|---|---|---|---|
| Number of Chicken | Titer of Neutralization Antibody | | | | | | |
| | <160 | 160 | 640 | 1280 | 2560 | 5120 | 10240 |
| 10 | 0 | 0 | 0 | 0 | 8 | 0 | 2 |

| | | | | | | | |
|---|---|---|---|---|---|---|---|
| (Note) Titer of neutralization antibody in control was less than 10 times. | | | | | | | |

### Example 3

CE primary culture cells were suspended in Eagle's MEM medium (pH 7.2) supplemented with 5% calf serum at a concentration of 1 x 10⁵ cells per 1 ml of the culture medium. The resulting culture medium was mixed with chick intrauterine PPV Nakano strain-derived KIII strain at m.o.i. of 0.05 and adjusted to 150 ml. A suspension culture was conducted in a 500 ml spinner vessel at 37°C for 6 days with stirring to allow for growth of PPV. Every day during culture, 10 ml of the culture were sampled and a culture solution was obtained after removal of cells with a 1 µm filter.

The viral content of these culture solutions was measured by 50% egg infectious dose (EID₅₀) using SPF developed hen's egg where those cells showing pock on the chorioallantoic membrane were considered to be infected with viruses. The results are shown in Table 5.

The viral content was measured in accordance with "Standard for Biological Preparation for Animals" (published by the Association of Biological Preparation for Animals on June 1993, page 139-141).

PPV was shown to grow by suspension culture of PPV-CE in accordance with the present invention.

**Table 5**

| Viral Growth in PPV-CE Suspension Culture | | | | | | | |
|---|---|---|---|---|---|---|---|
| | Viral Content (Log₁₀ EID₅₀/ml) | | | | | | |
| Suspension Culture | 0 | 1 | 2 | 3 | 4 | 5 | 6 (Day) |
| | 3.4 | 4.5 | 4.8 | 5.2 | 6.5 | 6.6 | 7.0 |

### Example 4

CE primary culture cells were suspended in Eagle's MEM medium (pH 7.2) supplemented with 5% calf serum at a concentration of 1 x 10⁵ cells per 1 ml of the culture medium. The resulting culture medium was mixed with attenuated ILTV-CE strain at m.o.i. of 0.1 and adjusted to 150 ml. A suspension culture was conducted in a 500 ml spinner vessel at 37°C for 4 days with stirring to allow for growth of ILTV. Every day during culture, 10 ml of the culture were sampled and a culture solution was obtained after removal of cells with a 1 µm filter.

The viral content of these culture solutions was measured by TCID₅₀ using CK cells where those cells showing CPE were considered to be infected with viruses. The results are shown in Table 6.

The culture solution on Day 4 was used as a live vaccine and administered by eye drops to 35 days old SPF chicken at 10^{4.7} TCID₅₀/0.1 ml and immunization was conducted for 2 weeks. After immunization, a titer of the live vaccine against chicken was measured by inoculating virulent ILTV NS-175 strain (0.1 ml), which has been adjusted to 100 times larger amount than the amount which induces disease in more than 50% of inoculated chicken, within the trachea of the immunized chicken. The results are shown in Table 7.

The viral content and the titer against chicken were measured in accordance with "Standard for Biological Preparation for Animals" (published by the Association of Biological Preparation for Animals on June 1993, page 168-172).

As is clear from the results shown in Table 7, the ILTV live vaccines prepared by suspension culture of ILTV-CE in accordance with the present invention exhibited a sufficient titer against chicken.

**Table 6**

| Viral Growth in ILTV-CE Suspension Culture | | | | | |
|---|---|---|---|---|---|
| | Viral Content (Log₁₀ TCID₅₀/ml) | | | | |
| Suspension Culture | 0 | 1 | 2 | 3 | 4 (Day) |
| | 3.8 | 4.5 | 5.1 | 5.9 | 5.7 |

**Table 7**

| Titer of ILTV Live Vaccine against Chicken | | |
|---|---|---|
| Number of Chicken Tested | Number of Chicken with Onset of Disease | Protection Rate against Onset of Disease (%) |
| 10 | 2 | 80 |

| | | |
|---|---|---|
| (Note) Protection rate against onset of disease (%) in control was 0%. | | |

## Claims

1. A process for growing a virus by infecting chick embryo (CE) primary culture cells with said virus and culturing said cells, characterized in that said culture is conducted by a suspension culture in a culture medium without adhesion of the cells to a solid phase.

2. The process of claim 1 wherein said suspension culture of CE primary culture cells is conducted in a culture tank without using a culture bottle, a hollow fiber or a microcarrier.

3. The process of claim 1 or 2 wherein said virus is one capable of infecting CE or CE primary culture cells.

4. The process of claim 3 wherein said virus is selected from the group consisting of infectious bursal disease virus (IBDV), avian reovirus (ARV), pigeon pox virus (PPV), fowl pox virus (FPV), avian infectious laryngotracheitis virus (ILTV), turkey herpesvirus, Marek's disease virus, Newcastle disease virus, avian infectious bronchitis virus, egg drop syndrome-1976 (EDS-76) virus, Ibaraki virus, bovine parainfluenza type 3 virus, rabies virus, rubella virus, mumps virus and influenza virus.
